# EUROPÄISCHE PATENTSCHRIFT

(11) **EP 1 180 980 B1**
(45) Veröffentlichungstag und Bekanntmachung des Hinweises auf die Patenterteilung: **29.03.2006**
(21) Anmeldenummer: 00936523.0
(22) Anmeldetag: 18.05.2000
(51) Int. Cl.: A61B 17/72

(54) **TIBIAMARKNAGEL**
INTRAMEDULLARY NAIL FOR THE TIBIA
CLOU MEDULLAIRE POUR TIBIA

(30) Priorität: 21.05.1999 AT 35999 U
(43) Veröffentlichungstag der Anmeldung: 27.02.2002
(73) Patentinhaber: I.T.S. Implantat-Technologie-Systeme GmbH, 8047 Graz (AT)
(72) Erfinder: HERTZ, Harald, A-1180 Wien (AT)
(74) Vertreter: Haffner, Thomas M.
(86) Internationale Anmeldenummer: PCT/AT2000/000139
(87) Internationale Veröffentlichungsnummer: WO 2000/071040

(56) Entgegenhaltungen:
- EP-A- 0 827 717
- WO-A-94/13219
- US-A- 4 781 181
- US-A- 5 472 444
- US-A- 5 713 901
- US-A- 5 713 902

## Beschreibung

Die Erfindung bezieht sich auf einen Marknagel, insbesondere Tibiamarknagel mit sich quer zum Nagel erstreckenden Durchbrechungen für die Aufnahme von Verriegelungsschrauben im proximalen und im distalen Bereich des Marknagels.

Marknägel und insbesondere Tibianagelsysteme werden zumeist unter Verwendung eines aus Titan gefertigten Tibianagels gebildet. Derartige Marknägel werden insbesondere bei transversalen Frakturen, Schräg- und Spiralfrakturen, Segment frakturen, Trümmerfrakturen, offenen Frakturen, und insbesondere auch Frakturen mit Knochenverlust oder pathologischen Frakturen eingesetzt.

Weitere Einsatzgebiete betreffen die Korrektur von Beinlängendifferenzen, Rekonstruktionen nach Tumorresektion, Pseudoarthrosen, Metaphysen und Epiphysenfrakturen. Bekannte Marknägel weisen im proximalen und distalen Bereich Möglichkeiten für eine Verriegelung auf, wobei für eine vollständige Verriegelung in aller Regel jeweils zwei Verriegelungsschrauben im proximalen und im distalen Bereich angeordnet werden. Bei der Anwendung wird zunächst die passende Nagellänge bestimmt und ein proximales Zielgerät mittels eines Adapters auf einem Nagel mit entsprechender Länge befestigt. In der Folge wird ein Gleithammer über eine Einschlagstange geschoben und diese mit dem Adapter verschraubt. Der Nagel wird in der Folge von Hand in das Eintrittsloch des proximalen Markraumes geführt und mit Gleithammerschlägen vorangetrieben. Im Falle eines Tibianagels soll die distale Positionierung idealerweise 1 bis 2 cm über dem Tibiotalarsytem liegen.

Für die proximale Verriegelung wird der Adapter zunächst festgezogen und die proximale Verriegelungsschraubenposition durch ein Doppelführungskanülensystem vorgegeben. In der Folge werden Bohrhülsen über eine Stichinzision auf die Kortikalis gebracht und eine entsprechende Bohrung vorgenommen. Wenn die hintere Kortikalis spürbar durchbohrt wurde, wird die korrekte Lage überprüft und an der Bohrerskalierung die erforderliche Schraubenlänge abgelesen. Nach Entfernen der inneren Bohrhülse kann eine entsprechende selbstschneidende Kortikalisschraube eingeschraubt werden. Für die distale Verriegelung werden die entsprechenden Bohrungen üblicherweise freihändig ausgeführt. Für eine vollständige Verriegelung ist es insbesondere im proximalen Bereich vorteilhaft, wenn die Bohrungen für die Aufnahme der Verriegelungsschraube mit einander schneidenden Achsen im Nagel angeordnet sind.

Die bekannten Nägel und insbesondere die bekannten Tibianägel werden insbesondere im Zusammenhang mit der Indikation einer Metaphysen- und Epiphysenfraktur nicht allen Situationen gerecht. Mit den am distalen Ende angeordneten Verriegelungsschrauben lassen sich nämlich nur derartige Knochensplitter erfolgreich korrekt positionieren, welche sich in diesem Bereich befinden, sodaß insbesondere kleine Knochensplitter am Knochenende, deren Fissur sich nur über eine geringe axiale Länge des Knochens erstreckt, mit derartigen Einrichtungen nicht erfaßt werden können. Die Erfindung zielt nun darauf ab, die universelle Verwendbarkeit eines Marknagels und insbesondere eines Tibiamarknagels auch auf Fälle zu erweitern, in welchen eine erfolgreiche Festlegung von Bruchstücken des Knochens beispielsweise nach Abscherungsbrüchen des Tibiarandes nicht ohne weiteres möglich ist und insbesondere den Heilungserfolg im Zusammenhang mit Epiphysenfrakturen zu verbessern.

Zur Lösung dieser Aufgabe besteht der erfindungsgemäße Marknagel im wesentlichen darin, daß die Achse wenigstens einer Durchbrechung im distalen Bereich zur Schaftachse im distalen Bereich geneigt verläuft. Dadurch, daß im distalen Bereich eine geneigte Durchbrechung vorgesehen ist, gelingt es eine Verriegelungs- oder gegebenenfalls Spannschraube in diesem Bereich so anzuordnen, daß auch kleinere Knochensplitter im Epiphysenbereich sicher positioniert und in ihrer korrekten Position gehalten werden können. Die Neigung dieser Durchbrechung im distalen Bereich zur jeweiligen Schaftachse erlaubt Schrauben mit nach hinten unten gerichteter Achse zu positionieren und auf diese Weise auch kleine Splitter zu erfassen. Bekannte Ausbildungen von Tibianägel weisen mehrere Abschnitte mit voneinander verschiedener und zueinander geneigter Achse auf. Insbesondere sind Ausbildungen bekannt, bei welchen Achsen des proximalen und des distalen, die Durchbrechungen für Verriegelungsschrauben aufweisenden Abschnitte des Marknagels zur Achse des mittleren Abschnittes in gleicher Richtung abgewinkelt bzw. geneigt angeordnet sind. Auch hier ist es wesentlich, daß die Achse wenigstens einer Durchbrechung im distalen Bereich zur Schaftachse im distalen Bereich geneigt verläuft. Bei konventionellen Tibianägeln liegen die Durchbrechungen im distalen Bereich üblicherweise so, daß sie trotz der Neigung der Achse des Nagels im distalen Bereich relativ zur Achse des Nagels im mittleren Bereich immer noch orthogonal zur Achse im mittleren Bereich orientiert sind.

Mit Vorteil ist die erfindungsgemäße Ausbildung so getroffen, daß die Neigung der Achse der Durchbrechung zwischen 65 und 80°, vorzugsweise etwa 75°, nach hinten gerichtet ist, wodurch sichergestellt ist, daß auch kleine Knochenabsplitterungen im Epiphysenbereich erfaßt werden können. Die Erfaßbarkeit derartiger kleiner Splitter wird noch dadurch verbessert, daß die Durchbrechung mit geneigter Achse dem distalen Ende des Marknagels benachbart angeordnet ist.

Eine weitere Verbesserung des erfindungsgemäßen Marknagels läßt sich dadurch erzielen, daß am proximalen Ende zusätzlich zu wenigstens zwei Durchbrechungen mit einander kreuzenden Achsen wenigstens eine weiter distal angeordnete Durchbrechung als Langloch mit in Richtung der Achse des Marknagels verlaufender längerer Achse der Durchbrechung vorgesehen ist. Eine derartige, als Langloch ausgebildete, zusätzliche Durchbrechung dient der Dynamisierung um mögliche Pseudoarthrosen zu verhindern. Neben der durch die beiden anderen Bohrungen nach Einsetzen der Verriegelungsschrauben erzielten Rotationsstabilität wird auf diese Weise eine axiale Beweglichkeit ermöglicht und es werden bei dieser Dynamisiserung Mikrobewegungen zugelassen, wodurch das Knochenwachstum angeregt werden kann.

Die Rotationsstabilität kann hiebei in bekannter Weise dadurch erzielt werden, daß wenigstens zwei der proximalen Durchbrechungen mit einander kreuzenden Achsen mit Verriegelungsschrauben verriegelt sind, wobei zum Schutz und zur Verminderung von Irritationen der Patellarsehne das proximale Ende des Tibianagels in bekannter Weise abgeschrägt ausgebildet sein kann.

Die am distalen Ende angeordnete, schräg nach hinten geneigt verlaufende Durchbrechung erlaubt es insbesondere bei Abscherungsbrüchen des Tibiarandes auch diese Knochenfragmente fixieren zu können. Eine besonders vorteilhafte Vereinfachung in der Handhabung läßt sich hiebei dadurch erzielen, daß die lichte Weite aller Durchbrechungen für Verriegelungsschrauben mit gleichem Durchmesser ausgebildet ist. Bei konventionellen Tibiamarknagelsystemen werden im distalen Bereich Schrauben mit geringerem Durchmesser als im proximalen Bereich eingesetzt.

Um nun sicherzustellen, daß derartige Verriegelungsschrauben bei jeweils geringstem Außendurchmesser auch die erforderlichen Kräfte und insbesondere die erforderliche Rotationsstabilität und die distale Verriegelung gewährleisten, ist mit Vorteil die Ausbildung so getroffen, daß die Verriegelungsschrauben in ihrem mittleren Bereich als gewindeloser Bolzenabschnitt mit der lichten Weite der Durchbrechungen entsprechendem Durchmesser ausgebildet sind. Derartige in ihrem mittleren Bereich als gewindeloser Bolzenabschnitt ausgebildete Verriegelungsschrauben weisen eine höhere Biegesteifigkeit und eine bessere Kraftaufnahme für quer zur Achse der Schraube gerichtet auftretende Kräfte gegenüber durchgehend ein Gewinde aufweisenden Schrauben auf, wobei die Verriegelung dann gelingt, wenn der Durchmesser des gewindelosen Bolzenabschnittes im wesentlichen der lichten Weite der Durchbrechungen entspricht.

Um nun bei Abscherungsbrüchen des Tibiarandes auch kleine Knochenfragmente in ihre gewünschte Position zu verspannen, ist mit Vorteil die Ausbildung so getroffen, daß die Schraube für die zur Achse des distalen Abschnittes geneigte Durchbrechung als Spannschraube ausgebildet ist, wobei sich eine derartige Spannschraube durch zwei Gewindeabschnitte mit unterschiedlichen Gewindesteigungen auszeichnet.

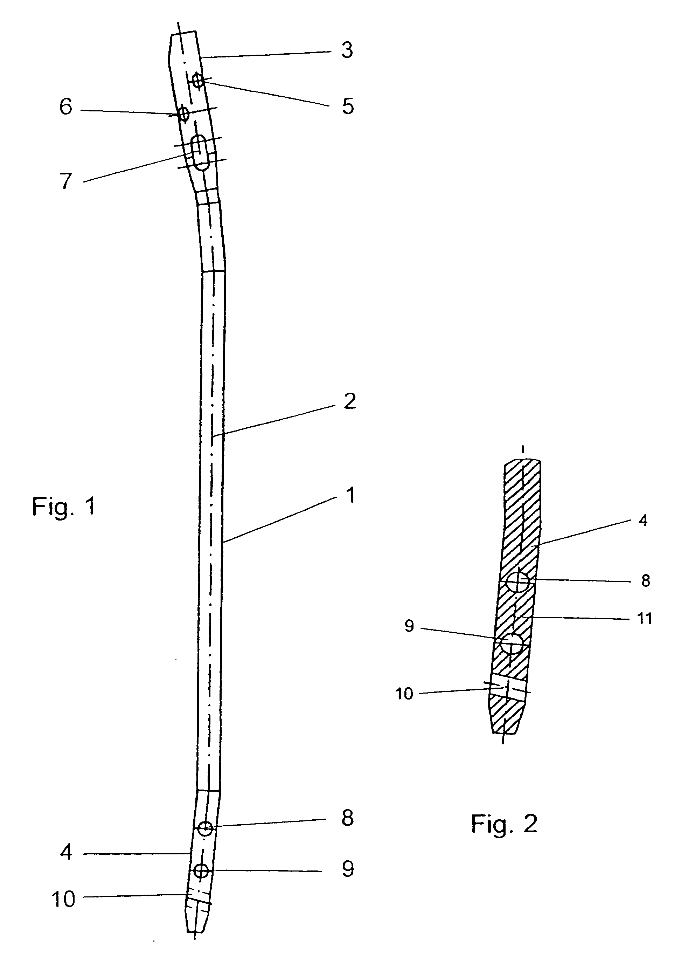

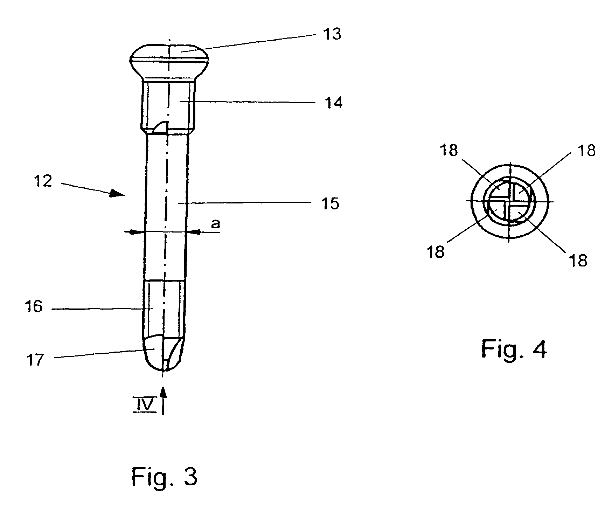

Die Erfindung wird nachfolgend anhand eines in der Zeichnung schematisch dargestellten Ausführungsbeispieles näher erläutert. In dieser zeigen Fig. 1 eine Seitenansicht eines erfindungsgemäßen Tibianagels, Fig. 2 einen Schnitt durch den distalen Endbereich des Tibianagels nach Fig. 1, Fig. 3 eine Ansicht einer Verriegelungs- oder Spannschraube und Fig. 4 eine Ansicht in Richtung des Pfeiles IV der Fig. 3 auf die Verriegelungsschraube bzw. Spannschraube.

In Fig. 1 ist ein Tibiamarknagel 1 dargestellt, dessen Achse strichpunktiert mit 2 bezeichnet ist. Die Achse des Tibiamarknagels 1 ist hiebei mehrfach gekröpft ausgebildet und in Richtung zum proximalen Ende 3 ebenso wie in Richtung zum distalen Ende 4 gleichsinnig geneigt zur Achse 2 im mittleren Bereich des Tibiamarknagels.

Das proximale Ende weist einander kreuzende Bohrungen 5 und 6 für die Rotationsverriegelung auf. Zusätzlich ist im proximalen Endbereich ein Langloch 7 vorgesehen, über welches eine weitere Verriegelungsschraube eingebracht werden kann, wobei aufgrund der Ausgestaltung der Durchbrechung als Langloch 7 hier eine axiale Bewegung des Tibiamarknagels 1 und damit eine Dynamisierung ermöglicht wird.

Das distale Ende 4 weist wiederum zwei Bohrungen 8 und 9 mit im wesentlichen orthogonal zur Achse 2 verlaufender Bohrungsachse für die Aufnahme von Verriegelungsschrauben auf. Im distalen Endbereich ist in Fig. 1 mit 10 eine nach hinten geneigte Bohrung angedeutet, welche im Schnitt in der Darstellung nach Fig. 2 deutlich ersichtlich ist.

In Fig. 2 ist diese Bohrung 10 unter einem Winkel von etwa 70° zur Achse 11 des distalen Bereiches 4 des Tibianagels geneigt angeordnet, sodaß sich insgesamt eine Gesamtneigung zur Achse 2 im mittleren Bereich des Tibianagels von etwa 65° ergibt. Durch die Bohrungen bzw. Durchbrechungen 5, 6, 7, 8, 9 und 10 können nun konventionelle Verriegelungsschrauben oder aber Spannschrauben geführt werden, um auf diese Weise eine sichere Verankerung des Tibianagels zu gewährleisten und gegebenenfalls zusätzlich Splitter erfolgreich zu fixieren. Eine für die Verwendung mit einem derartigen Tibiamarknagel nach den Fig. 1 und 2 geeignete Spann- bzw. Verriegelungsschraube ist in den Fig. 3 und 4 dargestellt.

In Fig. 3 ist eine Verriegelungsschraube 12 ersichtlich, welche im Anschluß an einen Schraubenkopf 13 einen ersten Gewindeabschnitt 14, einen mittleren gewindelosen Bolzenabschnitt 15 und einen endständigen Gewindeabschnitt 16 aufweist. Am freien Ende der Verriegelungsschraube ist im Anschluß an das Gewinde 16 ein Schneidkopf 17 vorgesehen, bei welchem, wie sich insbesondere aus Fig. 4 ergibt, zwischen Gewindeabschnitten Freiräume 18 für den Abtransport des geschnittenen Materials ausgebildet sind, um ein Einschrauben der Schraube im Knochensplitter bzw. die Kortikalis zu erleichtern.

Der mittlere Abschnitt 15 der Verriegelungsschraube ist hiebei gewindelos ausgebildet und weist einen Außendurchmesser a auf, welcher dem lichten Querschnitt der Durchbrechungen des Tibiamarknagels im wesentlichen entspricht. Um die Durchführung der Verriegelungsschrauben durch die Durchbrechungen zu ermöglichen, ist das endständige Gewinde mit einem Außendurchmesser ausgebildet, welcher maximal diesem Durchmesser a des gewindelosen Bereiches im mittleren Teil entspricht, wobei eine Festlegung im Knochen an der dem freien Ende gegenüberliegenden Seite durch das entsprechend größeren Durchmesser aufweisende Gewinde 14 gewährleistet ist. Wenn die Verriegelungsschraube 12 als Spannschraube eingesetzt werden soll, muß das Gewinde 14 mit geringerer Steigung als das Gewinde 16 ausgebildet sein, sodaß beim Einschrauben ein Anziehen von Knochensplittern in Richtung zum mittleren, gewindelos ausgebildeten Bereich 15 der Schraube 12 erfolgt.

## Patentansprüche

1. Tibiamarknagel (1) mit sich quer zum Nagel erstreckenden Durchbrechungen (5,6,7,8,9,10) für die Aufnahme von Verriegelungsschrauben (12) im proximalen (3) und im distalen (4) Bereich des Marknagels (1), **dadurch gekennzeichnet, dass** die Achse wenigstens einer Durchbrechung (10), welche dem distalen Ende (4) des Marknagels (1) benachbart angeordnet ist, zur Schaftachse (11) im distalen Bereich (4) geneigt verläuft, wobei die Neigung der Achse der Durchbrechung (10) zwischen 65 und 80°, vorzugsweise etwa 75°, nach hinten unten gerichtet ist.

2. Marknagel nach Anspruch 1, **dadurch gekennzeichnet, dass** am proximalen Ende (3) zusätzlich zu wenigstens zwei Durchbrechungen (5,6) mit einander kreuzenden Achsen wenigstens eine weiter distal angeordnete Durchbrechung (7) als Langloch mit in Richtung der Achse des Marknagels verlaufender längerer Achse der Durchbrechung (7) vorgesehen ist.

3. Marknagel nach Anspruch 1 oder 2, **dadurch gekennzeichnet, dass** wenigstens zwei der proximalen Durchbrechungen (5,6) mit einander kreuzenden Achsen mit Verriegelungsschrauben (12) verriegelt sind.

4. Marknagel nach Anspruch 1, 2 oder 3, **dadurch gekennzeichnet, dass** das proximale Ende (3) des Marknagels (1) abgeschrägt ausgebildet ist.

5. Marknagel nach einem der Ansprüche 1 bis 4, **dadurch gekennzeichnet, dass** die lichte Weite aller Durchbrechungen (5,6,7,8,9,10) für Verriegelungsschrauben (12) gleichen Durchmessers ausgebildet ist.

6. Marknagel nach einem der Ansprüche 1 bis 5, **dadurch gekennzeichnet, dass** die Verriegelungsschrauben (12) in ihrem mittleren Bereich (15) als gewindeloser Bolzenabschnitt mit der lichten Weite der Durchbrechungen (5, 6, 7, 8, 9, 10) entsprechendem Durchmesser ausgebildet sind.

7. Marknagel nach einem der Ansprüche 1 bis 6, **dadurch gekennzeichnet, dass** die Achsen des proximalen und des distalen, die Durchbrechungen aufweisenden Abschnittes (3,4) des Marknagels (1) zur Achse (2) des mittleren Abschnittes in gleicher Richtung abgewinkelt bzw. geneigt angeordnet sind.

8. Marknagel nach einem der Ansprüche 1 bis 7, **dadurch gekennzeichnet, dass** die Schraube für die zur Achse (11) des distalen Abschnittes (4) geneigte Durchbrechung (10) als Spannschraube ausgebildet ist.

## Revendications

1. Clou médullaire de tibia (1), comportant des percements (5, 6, 7, 8, 9, 10) dirigés perpendiculairement au clou pour recevoir des vis de verrouillage (12) dans la zone proximale (3) et la zone distale (4) du clou médullaire (1),
**caractérisé en ce que** l'axe d'au moins un percement (10), qui est disposé au voisinage de l'extrémité distale (4) du clou médullaire (1), a une direction inclinée vers l'axe de tige (11) dans la zone distale (4), l'inclinaison de l'axe du percement (10) étant dirigée vers l'arrière et vers le bas, entre 65° et 80°, de préférence environ 75°.

2. Clou médullaire suivant la revendication 1, **caractérisé en ce qu'**à l'extrémité proximale (3), en plus d'au moins deux percements (5, 6) comportant des axes se croisant l'un l'autre, au moins un autre percement (7) disposé en prolongement distal est prévu sous la forme d'un trou oblong comportant un axe plus long du percement (7) dirigé suivant l'axe du clou médullaire.

3. Clou médullaire suivant la revendication 1 ou 2, **caractérisé en ce qu'**au moins deux des percements proximaux (5, 6) comportant des axes se croisant l'un l'autre sont verrouillés avec des vis de verrouillage (12).

4. Clou médullaire suivant la revendication 1, 2 ou 3, **caractérisé en ce que** l'extrémité proximale (3) du clou médullaire (1) est réalisée biseautée.

5. Clou médullaire suivant l'une des revendications 1 à 4, **caractérisé en ce que** l'ouverture libre de tous les percements (5, 6, 7, 8, 9, 10) est réalisée pour des vis de verrouillage (12) de même diamètre.

6. Clou médullaire suivant l'une des revendications 1 à 5, **caractérisé en ce que** les vis de verrouillage (12) sont réalisées, dans leur zone centrale (15), sous la forme de parties de goujon non filetées, comportant un diamètre correspondant à l'ouverture libre des percements (5, 6, 7, 8, 9, 10).

7. Clou médullaire suivant l'une des revendications 1 à 6, **caractérisé en ce que** les axes de la partie proximale (3) et de la partie distale (4) du clou médullaire (1) présentant les percements sont disposés en formant un angle ou une inclinaison de même direction vers l'axe (2) de la partie centrale.

8. Clou médullaire suivant l'une des revendications 1 à 7, **caractérisé en ce que** la vis pour le percement (10) incliné vers l'axe (11) de la partie distale (4) est réalisée sous la forme d'une vis de tension.

## Claims

1. Tibial intramedullary nail (1) with pierces (5,6,7,8,9,10) extending across the nail for the accommodation of locking screws (12) in the proximal (3) and distal (4) areas of the intramedullary nail (1), **characterized in that** the axis of at least one of the pierces (10) arranged in close vicinity of the distal end (4) of the intramedullary nail (1) extends at an angle to the sheath axis (11) in the distal area (4), wherein the angle of the axis of the pierce (10) is between 65 and 80°, preferably approximately 75°, towards the rear and downwards.

2. Intramedullary nail according to claim 1, **characterized in that** at the proximal end (3) in addition to at least two pierces (5,6) with intersecting axes at least one additional distally arranged pierce (7) is provided as an elongated hole with a longer axis of the pierce (7) extending in the direction of the axis of the intramedullary nail.

3. Intramedullary nail according to claim 1 or 2, **characterized in that** at least two of the proximal pierces (5,6) with intersecting axes are locked together by locking screws (12).

4. Intramedullary nail according to one of the claims 1 through 3, **characterized in that** the proximal end (3) of the intramedullary nail (1) is designed at an angle.

5. Intramedullary nail according to one of the claims 1 through 4, **characterized in that** the inside width of all pierces (5,6,7,8,9,10) for locking screws (12) is designed with the same diameter.

6. Intramedullary nail according to one of the claims 1 through 5, **characterized in that** the locking screws (12) in their center area (15) are designed as a thread-less bolt section with a diameter corresponding to the inside width of the pierces (5,6,7,8,9,10).

7. Intramedullary nail according to one of the claims 1 through 6, **characterized in that** the axes of the proximal and the distal sections (3,4) of the intramedullary nail (1), which contain the pierces for the locking screws, are bent, or arranged at an angle, respectively, in the same direction to the axis (2) of the center section.

8. Intramedullary nail according to one of the claims 1 through 7, **characterized in that** the screw for the pierce (10) angled toward the axle (11) of the distal section (4) is designed as a clamping screw.
